Europäisches Patentamt

European Patent Office

Office européen des brevets

(11) Publication number: **0 173 926**
**A1**

## (12) EUROPEAN PATENT APPLICATION

(21) Application number: **85110608.8**

(22) Date of filing: **23.08.85**

(51) Int. Cl.⁴: **A 61 N 5/10**

(30) Priority: **07.09.84 US 648864**

(43) Date of publication of application:
**12.03.86 Bulletin 86/11**

(84) Designated Contracting States:
**DE FR GB SE**

(71) Applicant: **Siemens Aktiengesellschaft**
**Berlin und München Wittelsbacherplatz 2**
**D-8000 München 2(DE)**

(72) Inventor: **Spanswick, Keith A.**
**384 Summert Street**
**Manchester Massachusetts 01944(US)**

(54) Electron accelerator.

(57) An electron accelerator which comprises a gantry (10) an electron beam defining system (16) mounted at the gantry (10) and means for mounting the gantry (10) between a floor (24) and a ceiling (26) of a treatment room.

FIG 1

EP 0 173 926 A1

## BACKGROUND OF THE INVENTION

### Field of the Invention

The present invention relates to an electron accelerator. In particular, it relates to an electron accelerator for intra-operative therapy.

### Description of the Prior Art

An electron accelerator for radiation therapy is, for example, described in the Siemens brochure "Siemens, a total resource company for radiation therapy", MG/5020-008 SIQI85. Conventional electron accelerators like the aforementioned one are not very suitable for intra-operative radiation therapy. These accelerators and the associated patient treatment tables are so designed that the patient's head is always directed towards the gantry of the electron accelerator. This prevents from free access to the patient's head, especially for an anesthetist. Free access to the head however, is important during surgery.

## SUMMARY OF THE INVENTION

### Objects

It is an object of this invention to provide an improved electron accelerator which allows free access to a patient's head, in particular in surgery situations.

### Summary

According to this invention, an improved electron accelerator is provided which comprises:

a)   a gantry;

b)   an electron beam defining system mounted at the gantry; and

c)   means for mounting the gantry between a floor and a ceiling of a treatment room.

In conventional electron accelerators the gantry together with the electron beam defining system are mounted on an upright stand which is positioned on the floor of the treatment room.  For balancing purposes, also when using an additional counterweight close to the floor, the beam defining system has to be arranged relatively close to the stand.  This gives the electron accelerator a relatively compact shape which allows patient treatment especially in the abdomen area only with a patient lying with his head inside the gantry structure.

In contrast to the prior art the electron accelerator according to this invention provides a gantry which is mounted between the floor and the ceiling of a treatment room.  An upright stand is no longer necessary.  Mounting the gantry between the floor and the ceiling of the treatment room however, provides for a much better balanced gantry support.  The torque generated by the weights of gantry and

- 3 -

beam definining system mounted between the floor and ceiling can be considerably larger than that generated by a gantry and beam defining system mounted at a stand. Under these circumstances, the beam defining system can be positioned at a greater distance from the support. The gantry becomes less compact and a patient can be positioned with respect to the more remote beam defining system with his feet inside the gantry and his head outside the gantry. This makes the electron accelerator according to this invention particularly suitable for intra-operative radiation therapy in operation rooms. Another advantage is that by mounting the gantry between floor and ceiling the weak floor is no longer loaded with the whole weight of the electron accelerator.

In a preferred embodiment of the invention an electron accelerator the beam defining system of which includes an electron accelerating wave guide, an electron beam bending system and a power supply and control means, further comprises means for mounting the electron accelerating wave guide and the electron beam bending system at the gantry inside the treatment room and means for mounting the power supply and control means outside the treatment room. This measure provides for further weight reduction on the side of the gantry and beam defining system. The reduced weight further improves the balancing stability of the complete system.

In another preferred embodiment of the invention, the gantry comprises a bearing mounted between the floor and ceiling of the treatment room, means for rotating the beam defining system and the bearing and means for restricting the rotation to angles less than $360^{o}$. Due to that, the height of the rotational axis of the gantry above floor can be reduced towards the working height of a normal size human person. In particular, the restricting means restrict the rotation to angles in the range of approximately $\pm 90^{o}$ with respect to an electron beam which is perpendicularly directed to the floor of the treatment room.

- 4 -

The foregoing and other objects, features and advantages of the invention will be apparent from the following more particular description of a preferred embodiment of the invention, as illustrated in the accompanying drawings.

## BRIEF DESCRIPTION OF THE DRAWINGS

In the drawings:

Fig. 1 is a partial cross-section of an electron accelerator according to this invention;

Fig. 2 is a top view of an electron accelerator according to this invention mounted in an operation room; and

Fig. 3 is a diagram showing the rotation angles.

Throughout the drawings, like elements are referred to by like numbers.

## DETAILED DESCRIPTION OF THE PREFERRED EMBODIMENTS

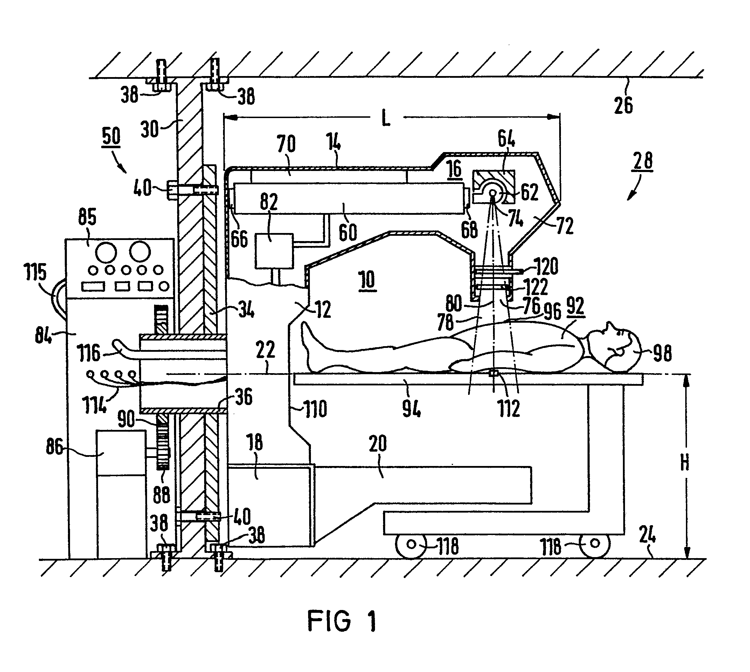

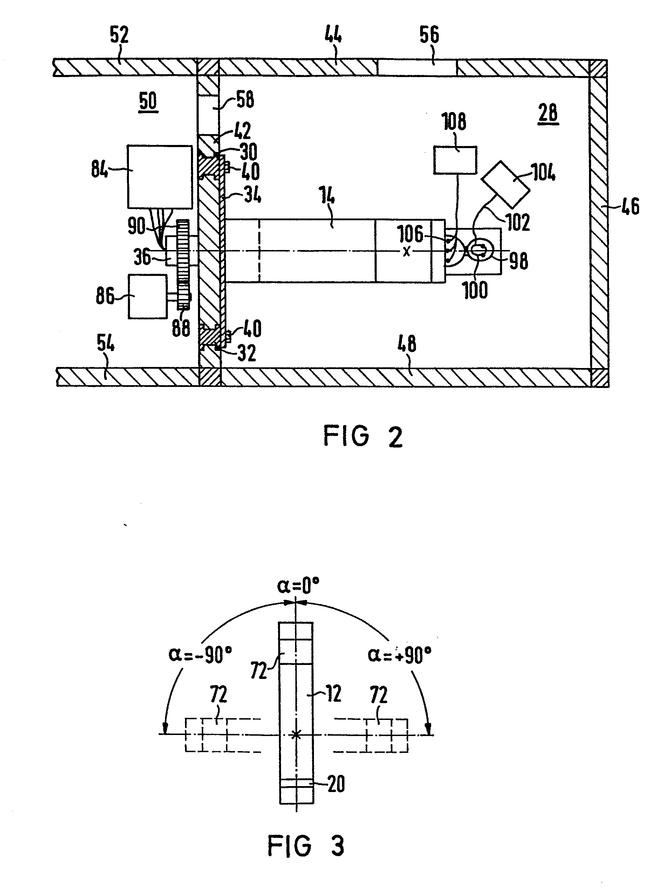

As illustrated in Figs. 1 and 2, the gantry 10 of the electron accelerator according to this invention comprises a vertical arm 12 and an elongated horizontal arm 14 at the upper end of the vertical arm 12. The elongated horizontal arm 14 contains an electron beam defining system 16. The lower end of the vertical arm 12 carries a counter weight 18 and an optional beam shield 20 for non-absorbed beam. The counterweight 18 and the optional beam shield 20 balance the weight of the elongated horizontal arm 14 with the electron beam defining system 16 with respect to a gantry rotational axis 22.

As also depicted in Figs. 1 and 2, the gantry 10 of the electron accelerator is mounted between the floor 24 and the ceiling 26 of a room 28, in particular operating room, by means of a first and second vertical beams 30, 32, a mounting plate 34, e.g. of steel, and a bearing 36 mounted in the mounting plate 34. The first and second beams 30, 32 are bolted with the floor 24 and the ceiling 26 by means of bolts 38. The mounting plate 34 is bolted with the first and second beams 30, 32 by means of bolts 40. Each beam 30, 32 has the shape of an I. The first and second beams 30, 32 and the mounting plate 34 are portions of a wall 42 of the room 28. The other walls of the room 28 are generally designated with 44, 46 and 48. The wall 42 is also a wall of a second room 50 adjoining to the room 28. In Fig. 2, two other walls of room 50 are indicated with 52 and 54. Two room doors are generally designated with 56 and 58.

According to Fig. 1, the electron beam defining system 16 comprises an electron accelerating wave guide 60, an electron accelerator vacuum envelope 62 and a bending magnet 64. The electron accelerating wave guide, which also includes an electron gun 66 and an electron exit window 68 is deposited in the interior of the elongated horizontal arm 14 of the gantry 10 by means of a supporting structure 17. The electron accelerator vacuum envelope 62 and the bending magnet 64 are arranged in the head 72 of the elongated horizontal arm 14 of the gantry 10 such that the exit window 74 of the electron accelerator vacuum envelope 62 is aligned towards an exit window 76 for the electron beam 78 at the lower end of the head 72. The center beam of the electron beam 78 is generally designated with 80. The element 82 is a circulator for preventing undesired reflections from the electron accelerating wave guide 16.

As previously mentioned mounting of a gantry between the floor and ceiling of a treatment room, as illustrated in Figs. 1 and 2, guarantees a much better balanced support then a normal stand of a conventional electron accelerator would do. Due to that, the length L of the elongated horizontal arm 14 of the gantry 10 in Figs. 1 and 2 can be chosen considerably longer than that of conventional arms. The arm length L can be further extended by additional weight reduction. In case of the embodiment of Figs. 1 and 2 all power supply and control means for the electron accelerating wave guide 16 and the electron beam bending system 62 and 64, also including the radio frequency power source, have been removed from the gantry and deposited outside the treatment room 28 in a special power supply equipment cabinet 84 and a control console 85 as indicated in Figs. 1 and 2. Both accessories can be positioned separately from each other. Also the gantry motor 86 together with the associated gear 88, 90 have been arranged outside the treatment room 28. According to Figs. 1 and 2 both the gantry motor 86, the power supply equipment cabinet 84 and the control console 85 are installed in the second room 50 close to the wall 42 which is common to both rooms 28 and 50.

By not making use of a stand alone leads to a weight reduction between 2500 and 3000 pounds. By positioning the power supply and control means outside the gantry another 1000 pounds can be saved. Therefore, according to this invention the weight of the whole electron accelerator can be reduced by up to 4000 pounds.

This weight reduction in combination with the special mounting procedure between floor and ceiling allows for a length L of the elongated horizontal arm 14 which is approximately 30 cm longer than that of conventional arms.

In a practical embodiment the elongated horizontal arm 14 according to Figs. 1 and 2 may have a length of approximately 208 cm. This allows a patient 92 lying on a patient treatment table 94 to become positioned with respect the electron window 76 of the head 72 such that the patient's abdomen 96 can be treated by the electron beam 78 with the patient's head 98 outside the gantry 10. This is important in case of intra-operative radiation therapy, when free access to the patient's head has to be guaranteed especially when the patient is under anesthetic. In addition also free access to the patient's chest is guaranteed. Both possibilities are indicated in Fig. 2 where the element 100 illustrates an anesthesia mask which is connected by means of a pipe 102 to an anesthetic apparatus 104. The patient's chest, however, is for example connected via electrodes 106 with patient monitoring apparatus 108 for e.g. ECG, blood pressure, etc. monitoring.

According to Fig. 1, the vertical arm 12 of the gantry 10 also comprises a recess 110 for the patient's feet. The recess is approximately 10 cm deep. This insures, that also relatively tall persons can be treated properly in the abdomen area with the patient's feet being arranged in the recess 110 inside the gantry and the patient's head being positioned outside the gantry.

In the embodiment of Figs. 1 and 2 the gantry 10 is designated for rotating within angles less than $360^o$. As illustrated in Fig. 3 the rotation is for example restricted to angles in the range of approximately $\pm 90^o$ with respect to an electron beam 78 which is perdicularly directed to the floor 24 of the treatment room. Under these circumstances the height of a rotational axis 22 of the gantry 10 above floor 24 can be reduced such that is approximately corresponds with the working height of a normal size human person. In the embodiment of Figs. 1 and 2 the rotation axis 22 of the gantry 10 is aligned with the isocenter 112 of the electron beam defining system 16. In practical operation situations the isocenter is approximately 1.1 meter above floor of the operation room 28. Restriction of the rotation angle can preferably be utilized by restricting the motor rotation. It can also be restricted by providing $\pm 90^o$ stops for the gantry.

In Figs. 1 and 2 all electrical connection cables between accessories inside the gantry and the power supply cabinet 84 outside the gantry are generally designated by 114. The pipe 116 connects a radio-frequency power source (not shown) inside the power supply equipment cabinet with the circulator 82 inside the gantry 10. Cable connections between the power supply equipment cabinet 84 and the control console 85 are indicated with the reference numeral 115. The patient treatment table 94 is movable by means of wheels 118. The elements 120 and 122 in the vicinity of the electron window 76 of the head 22 are a well known double scattering foil system and an electron dose chamber, respectively.

Having thus described the invention with particular reference to the preferred forms thereof, it will be obvious to those skilled in the art to which the invention pertains, after understanding the invention, that various changes and modifications may be made therein without departing from the spirit and scope of the invention as defined by the claims apended hereto.

WHAT IS CLAIMED IS:

1. An electron accelerator, comprising:

a) a gantry (10);

b) an electron beam defining system (16) mounted at the gantry; and

c) means (30, 32, 34) for mounting the gantry between a floor (24) and a ceiling (26) of a treatment room (28).

2. The electron accelerator according to claim 1, wherein the beam defining system comprises:

a) an electron accelerating waveguide (60);

b) an electron beam bending system (62, 64);

c) a power supply and control means (84, 85) for the electron accelerating waveguide and the electron beam bending system;

further comprising

d) means (70) for mounting the electron accelerating waveguide and the electron beam bending system at the gantry (10) inside the treatment room (28); and

e) means (24) for mounting the power supply and control means outside the treatment room (28).

- 11 -

3. The electron accelerator according to claim 1, wherein the gantry (10) comprises a bearing (36) mounted between the floor (24) and ceiling (26) of the treatment room (28).

4. The electron accelerator according to claim 3, wherein the means for mounting the gantry comprises a first and second beam (30, 32) each mounted between the floor (24) and ceiling (26) of the treatment room (28), and wherein the gantry bearing (36) is mounted between the first and second beams.

5. The electron accelerator according to claim 4, further comprising a mounting plate (34), said mounting plate being attached to the first and second beams (30, 32) and the gantry bearing (36) being mounted at the mounting plate (34).

6. The electron accelerator according to claim 5, wherein the mounting plate (34) is made of metal.

7. The electron accelerator according to claim 1, wherein the means (30, 32 34) for mounting the gantry (10) define at least a portion of a wall (42) of the treatment room (28).

8. The electron accelerator according to claim 3, wherein the gantry (10) further comprises

   a) means (86, 88, 90) for rotating the beam defining system (16) in the bearing (36); and

   b) means (86) for restricting the rotation to angles $(\alpha)$ less than $360^o$.

9. The electron accelerator according to claim 8, wherein the restricting means (86) restrict the rotation to angles ( $\alpha$ ) in the range of approximately $\pm 90^O$ with respect to an electron beam (78) which is perpendicularly directed to the floor of the treatment room.

10. The electron accelerator according to claim 9, wherein the gantry bearing (36) is mounted at a height (H) above the floor (24) of the treatment room which approximately corresponds with the working height of a normal size human person.

11. The electron accelerator according to claim 10, wherein the mounting height (H) of the gantry bearing (36) approximately corresponds with the working height of a surgeon at an operation table in an operation room (28).

12. The electron accelerator according to claim 10, wherein the gantry bearing (36) comprises a horizontal longitudinal rotation axis (22), said axis being aligned with the isocenter (112) of the electron beam defining system (16).

13. The electron accelerator according to claim 12, wherein the isocenter (112) is approximately 1.1 m above the floor (24) of the treatment room (28).

14. The electron accelerator according to claim 1, further comprising

　　　a) a patient treatment table (94); and

　　　b) means (118) for positioning the patient treatment table with respect to the beam defining system (16) such that a patient's abdomen (96) can be treated when the patient (92) is lying on the treatment table beneath the beam defining system with his head (98) outside the gantry.

15. The electron accelerator according to claim 14, wherein the gantry (10) comprises:

a) a vertical arm (12) having an upper end;

b) an elongated horizontal arm (14) containing the electron beam defining system (16) at the upper end of the vertical arm; and

c) a bearing (36) for mounting the vertical arm between floor (24) and ceiling (26) of the treatment room (28);

wherein the length L of the elongated horizontal arm (14) is longer than that of conventional arms.

16. The electron accelerator according to claim 14, wherein the length(L)of the elongated horizontal arm (14) is approximately 30 cm longer than that of conventional arms.

17. The electron accelerator according to claim 16, wherein the length of the elongated horizontal arm is approximately 208 cm.

18. The electron accelerator according to claim 15, wherein the vertical arm (12) comprises a recess for a patient's feet.

19. The electron accelerator according to claim 18, wherein the recess (110) is approximately 10 cm deep.

20. The electron accelerator according to claim 1, further being designated for being used for intra-operative radiation therapy.

FIG 1

FIG 2

FIG 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-2 753 397 (VARIAN ASSOCIATES INC.) * Figure 1; page 18, lines 27-30 * | 1 | A 61 N 5/10 |
| A | | 3,4 | |
| Y | US-A-4 198 565 (ONO) * Figure 5; column 2, line 61 - column 3, line 27 * | 1 | |
| A | | 2 | |
| A | L'ELECTRICITE ELECTRONIQUE MODERNE, vol. 1, no. 3, March/April 1971, pages 5-11, Paris, FR; A. BERARD: "Les accélérateurs de particules" * Page 10, figures 11,12 * | 2,3,8-11,14,15 | |
| A | MEDICAL PHYSICS, vol. 11, no. 2, March/April 1984, pages 105-128, Am. Assoc. Phys. Med., New York, US; C.J. KARZMARK: "Advances in linear accelerator design for radiotherapy" * Page 113, figure 14; page 115, figure 18 * | 1,2,9,15 | |

TECHNICAL FIELDS SEARCHED (Int. Cl.4)

A 61 B
A 61 N

-/-

The present search report has been drawn up for all claims

| Place of search THE HAGUE | Date of completion of the search 11-11-1985 | Examiner ONILLON C.G.A. |
|---|---|---|

European Patent
Office

**EUROPEAN SEARCH REPORT**

0173926

Application number

EP 85 11 0608

Page 2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | BROWN BOVERI REVIEW, vol. 70, no. 9/10, September/October 1983, pages 318-323, Baden, CH; H. VOGT: "Linear accelerator dynaray-CH: A central component of the BBC radiotherapy system" * Page 320, figure 4; page 322, figures 8,9 * | 1 | |
| | --- | | |
| A | DE-B-1 064 680 (SIEMENS-REINIGER-WERKE AG) * Figures 1,2 * | 1 | |
| | ----- | | |
| | | | TECHNICAL FIELDS SEARCHED (Int. Cl.4) |

The present search report has been drawn up for all claims

| Place of search THE HAGUE | Date of completion of the search 11-11-1985 | Examiner ONILLON C.G.A. |
|---|---|---|

EPO Form 1503. 03.82